# EUROPÄISCHE PATENTSCHRIFT

(11) **EP 1 512 773 B1**
(45) Veröffentlichungstag und Bekanntmachung des Hinweises auf die Patenterteilung: **22.09.2010**
(21) Anmeldenummer: 04090311.4
(22) Anmeldetag: 10.08.2004
(51) Int. Cl.: C25D 9/02, A61L 27/34, A61L 27/54

(54) **Mit Chitosan beschichteter metallischer Gegenstand sowie Verfahren zu dessen Hertsellung**
Chitosan-coated metallic device and method of preparation
Produit métallique revêtu de chitosan, et procédé de préparation

(30) Priorität: 15.08.2003 DE 10338110
(43) Veröffentlichungstag der Anmeldung: 09.03.2005
(73) Patentinhaber: Biomet Deutschland GmbH, 14167 Berlin (DE)
(72) Erfinder: Nies, Berthold, 64407 Fraenkisch-Crumbach (DE); Worch, Hartmut, 01187 Dresden (DE); Sewing, Andreas, 64283 Darmstadt (DE); Beutner, René, 01217 Dresden (DE); Schwarnweber, Dieter, 01324 Dresden (DE)
(74) Vertreter: Gross, Felix

(56) Entgegenhaltungen:
- EP-A- 1 308 177
- WO-A-96/02259
- WO-A-02/059395
- WO-A-02/080996
- DE-A- 19 724 233
- DE-A- 19 724 869
- US-A- 3 585 084
- US-A- 5 578 073

## Beschreibung

Die Erfindung betrifft einen metallischen Gegenstand mit einer stabilen Beschichtung aus Chitosan sowie ein Verfahren zu deren Herstellung. Durch die Einbeziehung von biologisch aktiven Komponenten in die Chitosanbeschichtung kann diese an verschiedenste Anwendungen angepasst und die Biokompatibilität entsprechend modifizierter Oberflächen erhöht werden.
Derartig beschichtete metallische Gegenstände bzw. Oberflächen sind interessant für die Medizin und Tiermedizin, z. B. für Implantate, aber auch für verschiedenste Bereiche der Biotechnologie.

Das Polysacharid Chitosan und Modifikationen dieser Substanz werden in dem Gebiet der Biomaterialien für die Beschichtung von Implantaten sowie zur Erzeugung von Scaffolds für das Tissue Engineering vielfältig eingesetzt. So beschreibt U.S. 5,578,073 ein Dip-Coating Verfahren zur Erzeugung von Chitosan-PVA-basierten Schichten auf PTFE-Oberflächen zur Erzielung die Blutgerinnung verhindernder Oberflächen durch Einbeziehung biologisch aktiver Komponenten in die Schicht.
WO 96/02259 nutzt zur Beschichtung von Implantatoberflächen im Knochenkontakt ebenfalls einen Dip-Coating Prozess und setzt zur Stimulation der Hartgewebereaktion der Chitosanlösung Polysacharide wie Heparin, Heparansulfat, Chondroitinsulfat oder Dermatansulfat zu.
Die Erzeugung von Scaffolds für das Tissue Engineering von Knochen wird von Zhang et al. (J. of Non-Cryst. Solids 282(2,3), 159-64) durch die Kombination von Chitosan mit β-Tricalciumphosphat realisiert.
Ähnliches beschreibt EP0555807, bei dem ein entsprechendes Scaffold durch Mischung eines Chitosan-Sols mit pulveriger anorganischer Knochensubstanz und zweiwertigen Kationen hergestellt wird.

Die CA-2219399, welche stärker auf Weichgewebeanwendungen ausgerichtet ist, beschreibt die Erzeugung monolithischer Hydrogele auf der Basis von Chitosan und realisiert die Formgebung ausgehend von einer sauren Lösung des Polymers durch Hydrolyse eines zugesetzten Amids und die dadurch realisierte Neutralisation der ursprünglich sauren Lösung.

Als Nachteil wird im Zusammenhang mit den oben genannten Verfahren, insbesondere zur Ausbildung von Schichten, vielfach deren nicht ausreichende Stabilität diskutiert.

Davon ausgehend kombiniert WO 2002080996 eine extrem widerstandfähige langzeitstabile DLC-Schicht, der die Barrierefunktion zugeordnet ist, mit einer kurzzeitstabilen Polymerschicht (z.B. aus Poly-Milchsäure), in die biologisch aktive Wirkstoffe eingebunden sind.

In EP 1308177A1 wird zur Stabilisierung einer über Dip-Coating erzeugten Chitosanschicht nach deren Trocknung eine Lagerung in Stabilisierungslösung praktiziert. Dabei wird über den pH-Wert dieser Lösung oder die Anwendung von kovalenten Vernetzungsagenzien bzw. über die Kombination beider Ansätze die Stabilität des Films erhöht. In einem letzten Prozessschritt wird der stabilisierte Film gewaschen.

Die lockere Struktur durch Dip-Coating bzw. Solvent-Casting erzeugter Chitosanfilme folgt auch aus Untersuchungen von Cruz et al. (Anal. Chem. 72 (2000), 680-86). Diese Autoren zeigen für den Transport von insbesondere positiv geladenen Metallionenkomplexen durch über Solvent-Casting erzeugte Chitosanfilme, dass die Diffusionskoeffizienten denen der ungehinderten Diffusion in wässrigen Medien vergleichbar sind.

Für zahlreiche Anwendungen ist deshalb eine einfache Prozessführung zur Erzeugung fest haftender, dichter, eine Barrierewirkung aufweisender Chitosanfilme auf Biomaterialoberflächen von großem Interesse.

Aufgabe der vorliegenden Erfindung war es daher, einen metallischen Gegenstand mit einer stabilen Beschichtung aus Chitosan zu schaffen, bei dem in einem Prozessschritt definiert dichte, fest haftende Schichten erzeugt werden können und die Art der Prozessführung die Möglichkeit zur Einbeziehung biologisch aktiver Komponenten in die Schichten gibt.

Diese Aufgabe wird gelöst durch einen Gegenstand aus Metall, auf dessen Oberfläche aus einer schwach sauren Lösung von Chitosan elektrochemisch gestützt eine Chitosanschicht erzeugt wird. Durch Zugabe biologisch aktiver Komponenten zur Chitosanlösung ist deren Einbau in die Schicht möglich. Durch die elektrochemisch gestützte Erzeugung der Schicht erhält diese eine dichte Struktur, ist fest haftend und wirkt als Barriereschicht zwischen dem metallischen Substrat und der biologischen Umgebung.

Erfindungsgemäß erfolgt die Erzeugung der Schichten in einem wässrigen Elektrolyten durch kathodische Polarisation des zu beschichtenden metallischen Gegenstandes im galvanostatischen Mode mit einer Stromdichte zwischen 0.1-20 mA/cm², vorzugsweise zwischen 2 und 8 mA/cm². Die frei wählbaren elektrochemischen Parameter sind die Stromdichte, das Endpotential und der zeitliche Ablauf der Polarisation, wobei die Polarisation entweder kontinuierlich oder gepulst durchgeführt werden kann.

Der metallische Gegenstand kann dabei aus beliebigen Metallen oder Legierungen wie aus allen als metallisches Biomaterial eingesetzten Werkstoffen bestehen. Der Metallische Gegenstand kann eine beliebig komplexe Geometrie aufweisen und eine beliebige Oberflächenmorphologie (z.B. Gesandstrahlt, Titan-Plasmabeschichtet) haben.

In die auf dem erfindungsgemäßen metallischen Gegenstand erzeugte Chitosanschicht kann durch Zugabe zur Beschichtungslösung ein Einbau biologisch aktiver Komponenten erfolgen. Für den gewünschten Fall der schnellen Freisetzung der biologisch aktiven Komponente aus einem locker strukturierten Chitosanfilm kann der Prozess der elektrochemisch gestützten Schichterzeugung mit entsprechend gewählten Parametern betrieben werden (vorzugsweise niedrige Stromdichte und niedriges Endpotential) und/oder erweitert werden durch einen Dip-Coating Prozessschritt oder eine an die elektrochemische Polarisation anschließende Lagerung der Schicht in der Beschichtungslösung ohne Polarisation.
Als biologisch aktive Komponenten kommen Struktur- oder Adhäsionsproteine oder eine davon abgeleitete Peptidstruktur in Frage. Bevorzugt sind Antibiotika, Glykosaminoglykane, Proteoglykane, Zytokine oder eine davon abgeleitete Struktur.

lst die Freisetzung der biologisch aktiven Komponente aus dem Chitosanfilm nicht erwünscht, kann diese vorteilhaft durch Bindung der biologisch aktiven Komponente an die Chitosanschicht verhindert werden. Eine solche Bindung kann ionischer oder kovalenter Natur sein oder biologische Bindungen vom Typ Biotin//Avidin/Strepavidin ausnutzen. Kovalente Bindung wird z. B. durch Vernetzung mit UV-Licht oder andere chemische Reaktionen erreicht.

Ein weiterer Gegenstand der Erfindung ist ein Verfahren zur Herstellung des metallischen mit Chitosan beschichteten Gegenstandes nach Anspruch 1.

Überraschend wurde festgestellt, dass in einer schwach sauren wässrigen Lösung von Chitosan in einer organischen oder anorganischen Säure eine lokale Anhebung des pH-Wertes infolge kathodischer Polarisation eines zu beschichtenden metallischen Gegenstandes die Ausbildung einer stabilen, dichten, fest haftenden Chitosanschicht zur Folge hat. Dies äußert sich im Anstieg des Zellpotentials während der Polarisation. Dieses Zellpotential erreicht als Funktion der gewählten Stromdichte oberhalb eines Grenzwertes im Bereich von Sekunden bis Minuten Werte > 100 V und ist damit Ausdruck für die entstandene dichte, einen hohen ohmschen Widerstand darstellenden Schicht. Vorzugsweise wird die elektrochemische Polarisation mit einem Zellpotential im Bereich von 20 bis 110 V durchgeführt.

Erfindungsgemäß werden die Bedingungen für die Schichterzeugung derart gewählt, dass mit einer 0.1 bis 5 %igen Lösung von Chitosan in 0.1 bis 5 %iger Säure, vorzugsweise 1 bis 2 %iger, gearbeitet wird. Als Säuren sind sowohl anorganische als auch organische Säuren verwendbar.
Vorzugsweise werden als organische Säuren Milchsäure, Essigsäure oder Glutaminsäure eingesetzt. Als anorganische Säuren werden bevorzugt Salzoder Salpetersäure verwendet.

Der so beschichtete Gegenstand kann, mit den üblichen nichtthermischen Verfahren wie Ethylenoxid oder Gamma-Bestrahlung sterilisiert werden. Die Auswahl des Sterilisierungsverfahrens richtet sich dabei wesentlich nach der Stabilität der in der Schicht enthaltenen biologisch aktiven Komponente. Falls keine unmittelbar anschließende Verwendung erfolgt, kann der sterilisierte Gegenstand bei kühlen Temperaturen (<10°C) und unter Lichtausschluss gelagert werden.

Eine denkbare medizinische Anwendungsmöglichkeit des erfindungsgemäßen beschichteten Gegenstandes ist die bakteriostatische Ausrüstung einer Implantatoberfläche für Knochenkontakt durch eine Chitosanschicht allein oder die Kombination der Chitosanschicht mit dem Einbau von die Zelladhäsion fördernden Peptidsequenzen oder Proteinen wie z.B. Kollagen Typ l.
Eine andere Anwendungsmöglichkeit ist die Beschichtung der von externen Fixateuren insbesondere im Bereich des Durchtritts durch die Haut mit einer bakteriostatisch wirkenden Chitosanschicht oder die Kombination einer solchen Schicht mit in sie eingebauten entzündungshemmenden Wirkstoffen.

Das erfindungsgemäße Verfahren grenzt sich durch folgende wesentliche Vorteile von bekannten Verfahren ab:
■ Hohe, über die Wahl der elektrochemischen Parameter definiert einstellbare Stabilität, Dicke und Struktur des erzeugten Chitosanfilms.
■ Aufgrund der Stabilität und Dichtheit des Chitosanfilmes wirkt dieser als effektive Barriere zwischen dem metallischen Biomaterial und dem Umgebungsgewebe im Sinne einer Verminderung des korrosiven Angriffs des metallischen Biomaterials durch Bestandteile des Umgebungsgewebes bzw. Körperflüssigkeiten.
■ Verminderte lonenfreisetzung aus dem metallischen Biomaterial senkt das Risiko von Irritationen des Umgebungsgewebes und/oder nachteiligen systemischen Einflüssen und erhöht damit die Biokompatibilität des Gesamtkonstruktes.
■ Verminderte lonenfreisetzung aus dem metallischen Biomaterial infolge der Barrierewirkung der dichten Chitosanbeschichtung erlaubt auch bei Problempatienten den Einsatz preiswerterer Metalle als Material für den metallischen Teil des Gesamtkonstruktes.
■ Möglichkeit der gezielten Beeinflussung des Freisetzungsverhaltens und damit der Bioverfügbarkeit biologisch aktiver Bestandteile der Chitosanbeschichtung durch definierte Einstellung der Struktur der Chitosanschicht über die Auswahl der elektrochemischen Parameter der Schichterzeugung.

Ein weiterer Gegenstand der Erfindung ist die Verwendung des chitosanbeschichteten Gegenstandes als Material für medizinische Implantate.

Anhand nachfolgender Ausführungsbeispiele wird die Erfindung näher erläutert. Dabei werden in den Beispielen 1 und 2 Schichten mittlerer Dicke auf einer Titanlegierung bzw. einem Implantatstahl erzeugt. Beispiel 3 dokumentiert die Erzeugung dickerer, stabilerer Schichten. Beispiel 4 variiert die Vorgehensweise bei der Schichterzeugung zu einer gepulsten Polarisation und Beispiel 5 veranschaulicht die Einbeziehung einer biologisch aktiven Komponente in den Schichtaufbau. Beispiel 6 beschreibt die Variante von Chitosanlösungen in anorganischen Säuren wie HCl.

### Ausführungsbeispiel 1:

Eine zylindrische Probe aus TiAl6V4 mit einem Durchmesser von 10 mm und einer Dicke von 3 mm wird geschliffen, oxidpoliert und mit Ethanol gewaschen.
Eine 1 % Lösung von Chitosan in 1 % Milchsäure wird durch Rühren über Nacht erzeugt.
Die zylindrische Probe wird elektrisch kontaktiert und zusammen mit einem Blech aus rostfreiem Stahl als Gegenelektrode in die Chitosanlösung getaucht.
Die elektrochemische Polarisation erfolgt galvanostatisch mit einer Stromdichte von 3 mA cm⁻² bis zu einer Zellspannung von 30 V. Bei dieser Polarisation wird die TiAl6V4-Probe als Katode geschaltet.
Unmittelbar nach Ende der Polarisation wird die TiAl6V4-Probe aus der Chitosanlösung entnommen und mit destilliertem Wasser abgespült.
Visuell zeigt die Probe eine glatte fest haftende Schicht. Sowohl am Lichtmikroskop als auch in der rasterelektronenmikroskopischen Untersuchung erweist sich die Schicht als vollständig und dicht. In der FTIR-Spektroskopie wird auf der Probenoberfläche homogen ein Chitosanspektrum nachgewiesen.

### Ausführungsbeispiel 2:

Wie in Beispiel 1 aber mit Edelstahl 316L als zu beschichtendes Material.

### Ausführungsbeispiel 3:

Wie in Beispiel 1 aber mit Stromdichte von 5 mA cm⁻² bis zur Zellspannung von 100 V.

### Ausführungsbeispiel 4:

Wie in Beispiel 1 aber mit gepulster Polarisation.

### Ausführungsbeispiel 5:

Eine 1%ige Lösung von Chitosan und 0,1% Tropokollagen in 1% Milchsäure wird durch Rühren über Nacht erzeugt. Ansonsten wie in Beispiel 1.

### Ausführungsbeispiel 6:

Eine zylindrische Probe aus TiAl6V4 mit einem Durchmesser von 10 mm und einer Dicke von 3 mm wird geschliffen, oxidpoliert und mit Ethanol gewaschen.
Eine 1 % Lösung von Chitosan in 1 % HCl wird durch Rühren über Nacht erzeugt.
Die zylindrische Probe wird elektrisch kontaktiert und zusammen mit einer Gegenelektrode aus Platin blech in die Chitosanlösung getaucht.
Die kathodische Polarisation erfolgt galvanostatisch mit einer Stromdichte von 5 mA cm⁻² bis zu einer Zellspannung von 45 V.
Unmittelbar nach Ende der Polarisation wird die TiA16V4-Probe aus der Chitosanlösung entnommen und mit destilliertem Wasser abgespült.

## Patentansprüche

1. Gegenstand aus Chitosan-beschichtetem Metall, **dadurch gekennzeichnet, dass** auf der Oberfläche des Metalls durch elektrochemische Polarisation in einer chitosanhaltigen Lösung eine dichte, eine Barrierewirkung aufweisende Chitosanschicht vorliegt.

2. Gegenstand nach Anspruch 1, **dadurch gekennzeichnet, dass** die elektrochemische Polarisation galvanostatisch mit einer Stromdichte im Bereich zwischen 0.1 und 20 mA cm⁻² durchgeführt wird und der Gegenstand als Kathode polarisiert wird.

3. Gegenstand nach Anspruch 1 oder 2, **dadurch gekennzeichnet, dass** die elektrochemische Polarisation bis zu einem Zellpotential im Bereich von 5 bis 150 V durchgeführt wird.

4. Gegenstand nach einem der Ansprüche 1 bis 3, **dadurch gekennzeichnet, dass** die chitosanhaltige Lösung eine Chitosankonzentration von 0.1 bis 5 % aufweist.

5. Gegenstand nach einem der Ansprüche 1 bis 4, **dadurch gekennzeichnet, dass** die chitosanhaltige Lösung eine wässrige, schwach saure Lösung mit einer organischen oder anorganischen Säure darstellt.

6. Gegenstand nach Anspruch 5, **dadurch gekennzeichnet, dass** die chitosanhaltige Lösung Milchsäure, Essigsäure oder Glutaminsäure enthält.

7. Gegenstand nach Anspruch 5, **dadurch gekennzeichnet, dass** die chitosanhaltige Lösung Salzsäure oder Salpetersäure enthält.

8. Gegenstand nach einem der Ansprüche 1 bis 7, **dadurch gekennzeichnet, dass** die Säurekonzentration im Bereich von 0.1 bis 5 % liegt.

9. Gegenstand nach einem der Ansprüche 1 bis 8, **dadurch gekennzeichnet, dass** die Chitosanbeschichtung zusätzlich eine oder mehrere biologisch aktive Komponenten enthält.

10. Gegenstand nach Anspruch 9, **dadurch gekennzeichnet, dass** mindestens eine der biologisch aktiven Komponenten ein Strukturprotein, ein Adhäsionsprotein oder davon abgeleitete Peptidstruktur ist.

11. Gegenstand nach Anspruch 9, **dadurch gekennzeichnet, dass** mindestens eine der biologisch aktiven Komponenten ein Antibiotikum darstellt.

12. Gegenstand nach Anspruch 9, **dadurch gekennzeichnet, dass** mindestens eine der biologisch aktiven Komponenten ein Glykosaminoglykan, Proteoglykan oder eine davon abgeleitete Struktur darstellt.

13. Gegenstand nach Anspruch 9, **dadurch gekennzeichnet, dass** mindestens eine der biologisch aktiven Komponenten ein Zytokin darstellt.

14. Gegenstand nach einem der vorhergehenden Ansprüche, **dadurch gekennzeichnet, dass** die biologisch aktiven Komponenten nach der Erzeugung der Chitosanschicht nachträglich an diese gebunden werden.

15. Verfahren zur Herstellung eines mit einer dichten, eine Barrierewirkung aufweisenden Chitosanschicht beschichteten metallischen Gegenstandes, **dadurch gekennzeichnet, dass** der metallische Gegenstand in einer chitosanhaltigen Lösung kathodisch polarisiert wird.

16. Verfahren nach Anspruch 15, **dadurch gekennzeichnet, dass** die elektrochemische Polarisation galvanostatisch mit einer Stromdichte im Bereich zwischen 0.1 und 20 mA cm⁻² durchgeführt wird.

17. Verfahren nach einem der Ansprüche 15 oder 16, **dadurch gekennzeichnet, dass** die elektrochemische Polarisation bis zu einem Zellpotential im Bereich von 5 bis 150 V durchgeführt wird.

18. Verfahren nach einem der Ansprüche 15 bis 17, **dadurch gekennzeichnet, dass** als chitosanhaltige Lösung eine wässrige, schwach saure Lösung mit einer organischen oder anorganischen Säure verwendet wird.

19. Verfahren nach Anspruch 18, **dadurch gekennzeichnet, dass** als organische Säure Milchsäure, Essigsäure oder Glutaminsäure eingesetzt wird.

20. Verfahren nach Anspruch 18, **dadurch gekennzeichnet, dass** als anorganische Säure Salzsäure oder Salpetersäure eingesetzt wird.

21. Verfahren nach einen der Ansprüche 15 bis 20, **dadurch gekennzeichnet, dass** zur chitosanhaltigen Lösung eine oder mehrere biologisch aktive Komponenten zugesetzt werden.

22. Verfahren nach Anspruch 21, **dadurch gekennzeichnet, dass** als biologisch aktive Komponenten ein Strukturprotein, Adhäsionsprotein oder eine davon abgeleitete Peptidstruktur zugesetzt wird.

23. Verfahren nach Anspruch 21, **dadurch gekennzeichnet, dass** als biologisch aktive Komponente ein Antibiotikum zugesetzt wird.

24. Verfahren nach Anspruch 21, **dadurch gekennzeichnet, dass** als biologisch aktive Komponenten ein Glykosaminoglykan, Proteoglykan oder eine davon abgeleitete Struktur zugesetzt wird.

25. Verfahren nach Anspruch 21, **dadurch gekennzeichnet, dass** als biologisch aktive Komponente ein Zytokin zugesetzt wird.

26. Verwendung des chitosanbeschichteten Gegenstandes nach einem der Ansprüche 1 bis 14 als Material für medizinische Implantate.

## Claims

1. Article made from a chitosan-coated metal, **characterized in that** a dense chitosan layer having a barrier effect is present on the surface of the metal by an electro-chemical polarisation in a chitosan containing solution.

2. Article according to claim 1, **characterized in that** the electro-chemical polarisation is carried out in a galvanostatic manner with a current density in the range of 0.1 and 20 mA cm⁻² and the article is polarized as cathode.

3. Article according to claim 1 or 2, **characterized in that** the electro-chemical polarisation is carried out up to a cell potential in the range of 5 to 150 V.

4. Article according to any of claims 1 to 3, **characterized in that** the chitosan containing solution has a chitosan concentration of 0.1 to 5 %.

5. Article according to any of claims 1 to 4, **characterized in that** the chitosan containing solution makes up an aqueous, weakly acidic solution with an organic or inorganic acid.

6. Article according to claim 5, **characterized in that** the chitosan containing solution contains lactic acid, acetic acid or glutamic acid.

7. Article according to claim 5, **characterized in that** the chitosan containing solution contains hydrochloric acid or nitric acid.

8. Article according to any of claims 1 to 7, **characterized in that** the acid concentration is in the range of 0.1 to 5 %.

9. Article according to any of claims 1 to 8, **characterized in that** the chitosan coating additionally contains one or several biologically active components.

10. Article according to claim 9, **characterized in that** at least one of the biologically active components is a structural protein, an adhesion protein or a peptide structure derived therefrom.

11. Article according to claim 9, **characterized in that** at least one of the biologically active components makes up an antibiotic.

12. Article according to claim 9, **characterized in that** at least one of the biologically active components makes up a glycosaminoglycan, proteoglycan or a structure derived therefrom.

13. Article according to claim 9, **characterized in that** at least one of the biologically active components makes up a cytokine.

14. Article according to any of the preceding claims, **characterized in that** the biologically active components are subsequently bound to the chitosan layer after this layer has been produced.

15. Method for producing a metallic article being coated with a dense chitosan layer having a barrier effect, **characterized in that** the metallic article is cathodically polarized in a chitosan containing solution.

16. Method according to claim 15, **characterized in that** the electro-chemical polarisation is carried out in a galvanostatic manner with a current density in the range of 0.1 and 20 mA cm⁻²_{.}

17. Method according to claim 15 or 16, **characterized in that** the electro-chemical polarisation is carried out up to a cell potential in the range of 5 to 150 V.

18. Method according to any of claims 15 to 17, **characterized in that** an aqueous, weakly acidic solution with an organic or inorganic acid is used as chitosan containing solution.

19. Method according to claim 18, **characterized in that** lactic acid, acetic acid or glutamic acid are used as organic acid.

20. Method according to claim 18, **characterized in that** hydrochloric acid or nitric acid are used as inorganic acid.

21. Method according to any of claims 15 to 20, **characterized in that** one or several biologically active components are added to the chitosan containing solution.

22. Method according to claim 21, **characterized in that** a structural protein, adhesion protein or a peptide structure derived therefrom is added as biologically active component.

23. Method according to claim 21, **characterized in that** an antibiotic is added as biologically active component.

24. Method according to claim 21, **characterized in that** a glycosaminoglycan, proteoglycan or a structure derived therefrom is added as biologically active component.

25. Method according to claim 21, **characterized in that** a cytokine is added as biologically active component.

26. Use of the chitosan coated article according to any of claims 1 to 14 as material for medical implants.

## Revendications

1. Objet en métal revêtu de chitosane, **caractérisé en ce qu'**une couche de chitosane dense, présentant un effet de barrière, se trouve sur la surface de métal par polarisation électrochimique dans une solution contenant du chitosane.

2. Objet selon la revendication 1, **caractérisé en ce que** la polarisation électrochimique est réalisée de manière galvanostatique avec une densité de courant dans l'étendue entre 0,1 et 20 mA cm⁻² et l'objet est polarisé en tant que cathode.

3. Objet selon la revendication 1 ou 2, **caractérisé en ce que** la polarisation électrochimique est réalisée jusqu'à un potentiel de cellule dans l'étendue de 5 à 150 V.

4. Objet selon l'une quelconque des revendications 1 à 3, **caractérisé en ce que** la solution contenant du chitosane présente une concentration en chitosane de 0,1 à 5%.

5. Objet selon l'une quelconque des revendications 1 à 4, **caractérisé en ce que** la solution contenant du chitosane est une solution aqueuse, faiblement acide, contenant un acide organique ou inorganique.

6. Objet selon la revendication 5, **caractérisé en ce que** la solution contenant du chitosane contient de l'acide lactique, acétique ou glutamique.

7. Objet selon la revendication 5, **caractérisé en ce que** la solution contenant du chitosane contient de l'acide chlorhydrique ou de l'acide nitrique.

8. Objet selon l'une quelconque des revendications 1 à 7, **caractérisé en ce que** la concentration en acide se situe dans l'étendue de 0,1 à 5%.

9. Objet selon l'une quelconque des revendications 1 à 8, **caractérisé en ce que** le revêtement de chitosane contient en outre un ou plusieurs composants biologiquement actifs.

10. Objet selon la revendication 9, **caractérisé en ce qu'**au moins un des composants biologiquement actifs est une protéine de structure, une protéine d'adhésion ou une structure peptidique dérivée de celles-ci.

11. Objet selon la revendication 9, **caractérisé en ce qu'**au moins un des composants biologiquement actifs représente un antibiotique.

12. Objet selon la revendication 9, **caractérisé en ce qu'**au moins un des composants biologiquement actifs représente un glycosaminoglycane, un protéoglycane ou une structure dérivée de ceux-ci.

13. Objet selon la revendication 9, **caractérisé en ce qu'**au moins un des composants biologiquement actifs représente une cytokine.

14. Objet selon l'une quelconque des revendications précédentes, **caractérisé en ce que** les composants biologiquement actifs sont liés ultérieurement, après la production de la couche de chitosane, à celle-ci.

15. Procédé pour la production d'un objet métallique revêtu d'une couche de chitosane dense, présentant un effet de barrière, **caractérisé en ce que** l'objet métallique est polarisé cathodiquement dans une solution contenant du chitosane.

16. Procédé selon la revendication 15, **caractérisé en ce que** la polarisation électrochimique est réalisée de manière galvanostatique avec une densité de courant dans l'étendue entre 0,1 et 20 mA cm⁻².

17. Procédé selon l'une quelconque des revendications 15 ou 16, **caractérisé en ce que** la polarisation électrochimique est réalisée jusqu'à un potentiel de cellule dans l'étendue de 5 à 150 V.

18. Procédé selon l'une quelconque des revendications 15 à 17, **caractérisé en ce qu'**on utilise, comme solution contenant du chitosane, une solution aqueuse, faiblement acide, contenant un acide organique ou inorganique.

19. Procédé selon la revendication 18, **caractérisé en ce qu'**on utilise, comme acide organique, de l'acide lactique, acétique ou glutamique.

20. Procédé selon la revendication 18, **caractérisé en ce qu'**on utilise, comme acide inorganique, de l'acide chlorhydrique ou nitrique.

21. Procédé selon l'une quelconque des revendications 15 à 20, **caractérisé en ce qu'**on ajoute un ou plusieurs composants biologiquement actifs à la solution contenant du chitosane.

22. Procédé selon la revendication 21, **caractérisé en ce qu'**on ajoute, comme composant biologiquement actif, une protéine de structure, une protéine d'adhésion ou une structure peptidique dérivée de celles-ci.

23. Procédé selon la revendication 21, **caractérisé en ce qu'**on ajoute, comme composant biologiquement actif, un antibiotique.

24. Procédé selon la revendication 21, **caractérisé en ce qu'**on ajoute, comme composant biologiquement actif, un glycosaminoglycane, un protéoglycane ou une structure dérivée de ceux-ci.

25. Procédé selon la revendication 21, **caractérisé en ce qu'**on ajoute, comme composant biologiquement actif, une cytokine.

26. Utilisation de l'objet revêtu de chitosane selon l'une quelconque des revendications 1 à 14 comme matériau pour des implants médicaux.
